# EUROPEAN PATENT APPLICATION

(11) **EP 4 703 416 A1**
(43) Date of publication of application: **04.03.2026**
(21) Application number: 23939490.1
(22) Date of filing: 26.05.2023
(51) Int. Cl.: C08J 11/12

(54) **METHOD FOR RECOVERING RESIN DECOMPOSITION GAS, METHOD FOR PRODUCING REUSABLE LIQUEFIED GAS, AND APPARATUS FOR RECOVERING RESIN DECOMPOSITION GAS**

(71) Applicant: The Japan Steel Works, Ltd., Tokyo 141-0032 (JP)
(72) Inventor: SAGA, Daigo, Tokyo 141-0032 (JP)
(74) Representative: Simmons & Simmons LLP (Munich)
(86) International application number: PCT/JP2023/019797
(87) International publication number: WO 2024/247009

(57) **Abstract**

The present invention improves an operation rate of a recycling system for treating a resin. In the present disclosure, gas that solidifies when cooled to the final temperature is recovered as a liquid during a step of cooling to an intermediate temperature higher than the final temperature. Meanwhile, gas that does not liquefy at the intermediate temperature is liquefied and recovered by cooling from the intermediate temperature to the final temperature.

## Description

### TECHNICAL FIELD

The present invention relates to a technique of collecting resin decomposition gas, a technique of producing recyclable liquefied gas, and a resin decomposition gas collector, and relates to a technique effectively applied to, for example, a technique of liquefying and collecting a plurality of gases generated from thermal decomposition of a resin.

### BACKGROUND ART

Japanese Patent Application Laid-open Publication No. 2005-349838 (Patent Document 1) and Japanese Patent Application Laid-open Publication No. 2008-302555 (Patent Document 2) describe a technique of discharging gas that is generated inside an extruder when a resin is thermally decomposed under use of the extruder, to the outside through a vent provided in the extruder.

### RELATED ART DOCUMENTS

### PATENT DOCUMENTS

Patent Document 1: Japanese Patent Application Laid-open Publication No. 2005-349838
Patent Document 2: Japanese Patent Application Laid-open Publication No. 2008-302555

### SUMMARY OF THE INVENTION

### PROBLEMS TO BE SOLVED BY THE INVENTION

In recent years, an approach that is called "ESG" has attracted attention.

The "ESG" represents "Environment", "Social", and "Governance", and is known as the following approach.
(1) Approach to environmental problems of environment such as global warming and environmental pollution
(2) Improvement in work environment, Care for human rights, and Contribution to local activities and so forth
(3) Active information disclosure, Legal compliance, Setting of an outside board member and so forth

What is important in the approach "ESG" is that it is necessary to take a care for not only its own company but also parties concerned (stakeholders) such as customers, shareholders, correspondents, and local communities, and this is a point clearly positioned in the management policy and needs to be handled as a core business.

Management with the "ESG" to be considered as the core business is called "ESG management". An exemplified merit of handling the "ESG management" for companies is to provide a new business chance to sophisticate risk management and gather human resources and funds, leading to an improvement in enterprise value.

In the approach "ESG", when attention is paid to the approach to the environmental problems, it is important to achieve circular economy to solve the environmental problems. And, in order to achieve the circular economy, it is useful to, for example, take the recycling as much as possible.

Regarding this point, for example, plastic products are used in a very of wide range of fields, and its use amount is enormous. On the other hand, a process of discarding the plastic products costs much, and flow out of components of the plastic products, when being left under natural environments, to rivers and oceans to affect environments is also a problem. Therefore, in order to achieve the environment-friendly circular economy through the approach "ESG", it is necessary to establish a system for efficiently recycling the plastic products. Thus, it has been studied that a resin which is a component of plastic is effectively used by recycling it. In particular, it is desired to improve a utilization rate of a recycle system that processes a typified resin of the discarded plastics.

Note that "recycle" in the specification is a concept in a wide range including, for example, not only a mode of thermally decomposing a resin configuring the discarded product to synthesize a resin as a raw material again but also a mode of recycling a component as a fuel, generated by thermally decomposing the discarded resin.

### MEANS FOR SOLVING THE PROBLEMS

A method of collecting resin decomposition gas in one embodiment includes a step (a) of liquefying a first decomposition gas of the first decomposition gas and a second decomposition gas generated by thermally decomposing a resin, and a step (b) of liquefying the second decomposition gas after the step (a).

A method of collecting resin decomposition gas in one embodiment includes a step of separately liquefying and collecting a plurality of gases by using a difference in a boiling point among the plurality of gases generated by thermally decomposing a resin.

A method of producing recyclable liquefied gas in one embodiment includes a step (a) of liquefying a first decomposition gas of the first decomposition gas and a second decomposition gas generated by thermally decomposing a resin, and a step (b) of liquefying the second decomposition gas after the step (a).

A method of producing recyclable liquefied gas in one embodiment includes a step of separately liquefying and collecting a plurality of gases by using a difference in a boiling point among the plurality of gases generated by thermally decomposing a resin.

A resin decomposition gas collector in one embodiment includes a first cooler configured to liquefy a first decomposition gas to be collected of the first decomposition gas and a second decomposition gas generated by thermally decomposing a resin, and a second cooler configured to liquefy the second decomposition gas to be collected.

A resin decomposition gas collector in one embodiment includes a plurality of liquefying/collecting portions configured to separately liquefy and collecting a plurality of gases by using a difference in a boiling point among the plurality of gases generated by thermally decomposing a resin.

### EFFECTS OF THE INVENTION

According to one embodiment, a utilization rate of a recycle system that processes a resin can be improved.

### BRIEF DESCRIPTIONS OF THE DRAWINGS

FIG. 1 is a block diagram for explaining a conceptual structure of a recycle system.
FIG. 2 is a block diagram illustrating one example of an embodied mode of a basic idea.
FIG. 3 is a block diagram illustrating another example of the embodied mode of the basic idea.
FIG. 4 is a diagram illustrating a structure of a recycle system in the embodied mode.
FIG. 5 is a diagram illustrating an example of a structure of a resin decomposition gas collector.
FIG. 6 is a table indicating melting points and boiling points of hydrocarbon compounds of many types.
FIG. 7 is a diagram illustrating a recycle system including a resin decomposition gas collector in a first modification example.

### DETAILED DESCRIPTION OF PREFERRED EMBODIMENTS

The same components are denoted by the same reference symbols throughout all the drawings for describing the embodiments, and the repetitive description thereof will be omitted. Note that even a plan view may be hatched so as to make the drawings easy to see.

### <Study on Improvement>

First, a related art will be described, and then, a margin to be improved in the related art will be described.

The "related art" in the present specification is not a publicly-known technique but a technique having a problem found by the present inventors and a premise technique of the present invention.

For example, a recycle system that recycles a resin configuring plastic includes: a heat processor configured to thermally decompose a resin; a tank configured to store a decomposition product discharged from the heat processor; and a cooler configured to liquefy decomposition gas discharged from the heat processor. For example, while an extruder is used as the heat processor, a heat exchanger is used as the cooler.

When a resin typified by waste plastics is introduced to the extruder, the resin is thermally decomposed inside the extruder. As a result, from the extruder, gas (decomposition gas), liquid (melt), solid, and so forth are discharged because of the resin (raw material) and its decomposition reaction mode. Here, the solid and the liquid are discharged to the tank and collected. On the other hand, the decomposition gas can be collected as gas, but becomes difficult to be handled, as a result of low density and large volume. In consideration of this, in general, the decomposition gas is desirably collected as liquid. Therefore, the decomposition gas discharged from the extruder is cooled by the heat exchanger and then collected.

Here, normally, there are a plurality of types of decomposition gases generated by thermally decomposing the resin. Therefore, among the decomposition gases of the plurality of types, a decomposition gas with a melting point lower than a cooling temperature in the case of the heat exchanger can be liquified by the heat exchanger and then collected. By contrast, among the decomposition gases of the plurality of types, a decomposition gas with a melting point higher than the cooling temperature in the case of the heat exchanger is solidified when being cooled to the cooling temperature by the heat exchanger. If the decomposition gas is solidified as described above, there is a risk that is clogging of a passage for the decomposition gas inside the heat exchanger. In this case, the recycle system cannot continuously operate. As a result, this leads to a decrease in a utilization rate of the recycle system.

The following is detail description.

Consider that a polyolefin resin is introduced to the extruder for thermal decomposition. For example, when the heating temperature of the polyolefin resin is set at 400°C, alkanes each with a boiling point equal to or higher than 400°C among chain saturated hydrocarbons (alkanes) generated by the thermal decomposition of the polyolefin resin are alkanes each with a carbon number larger than 25. Therefore, the alkanes each with the carbon number larger than 25 are present with a solution state inside the extruder. On the other hand, alkanes each with a carbon number equal to or smaller than 25 is present with a gaseous state inside the extruder. Here, it is assumed that a cooling temperature of the heat exchanger for collecting the generated decomposition gas is 15°C. As for each melting point of the gasified alkanes each with the carbon number equal to or smaller than 25, a melting point of pentadecane with a carbon number of 15 is 9.9°C, and a melting point of hexadecane with a carbon number of 16 is 18°C.

Therefore, since a melting point of an alkane with a carbon number smaller than the carbon number of 15 of pentadecane is lower than the cooling temperature (15°C) of the heat exchanger, the alkane can be liquified and collected by the heat exchanger. By contrast, since a melting point of an alkane with a carbon number larger than the carbon number of 16 of hexadecane is higher than the cooling temperature of the heat exchanger, the alkane is undesirably solidified inside the heat exchanger. Therefore, when it is attempted that the gasified decomposition gas is liquified by the heat exchanger and then collected, an alkane with a melting point higher than the temperature of the heat exchanger among alkanes obtained by the thermal decomposition at 400°C is solidified, thereby causing the risk that is the clogging of the passage for the decomposition gas inside the heat exchanger. In this case, the recycle system cannot continuously operate. As a result, this leads to the decrease in the utilization rate of the recycle system.

As described above, the related art has a margin to be improved in view of suppression of the decrease in the utilization rate of the recycle system. Thus, in the present embodiment, a devisal is made to overcome the margin to be improved in the related art. A technical idea with this devisal in the present embodiment will be described below.

### <Basic Idea in Embodiment>

The basic idea in the present embodiment is an idea of using a difference in the boiling point among the plurality of gases produced by thermally decomposing the resin to separately liquify and collect the plurality of gases. In other words, the basic idea is an idea of liquifying and collecting a part of gases included in the plurality of gases and then collecting it by not collectively cooling the plurality of gases to a final temperature but cooling the plurality of gases to an intermediate temperature higher than the final temperature, and then, liquifying and collecting the other gases included in the plurality of gases, not liquified at the intermediate temperature by cooling the other gases to the final temperature. That is, the basic idea is an idea of collecting, as a liquid state, a gas that is solidified when being cooled to the final temperature, at a stage of cooling to the intermediate temperature higher than the final temperature, while liquifying and collecting a gas that is not liquified at the intermediate temperature, by cooling this gas from the intermediate temperature to the final temperature.

According to this basic idea, the gas solidification in the heat exchanger can be avoided, and, as a result, the clogging of the gas passage due to deposition of the solidified gas can be suppressed. Therefore, according to the basic idea, failure of the continuous operation of the recycle system due to the clogging of the gas passage can be prevented, and, as a result, the decrease in the utilization rate of the recycle system can be suppressed.

The suppression of the gas solidification by the basic idea can be explained below.

For example, it is assumed that a decomposition gas G1 and a decomposition gas G2 are included in the above-described plurality of gases. Here, it is assumed that a boiling point of the decomposition gas G1 is a temperature T1 while a boiling point of the decomposition gas G2 is a temperature T2 lower than the temperature T1. Also, it is assumed that a melting point of the decomposition gas G1 is a temperature T3 lower than the temperature T2 while a melting point of the decomposition gas G2 is a temperature T4 lower than the temperature T3.

First, discuss a case without the application of the basic idea in which the decomposition gas G1 and the decomposition gas G2 are collectively cooled to the final temperature lower than the temperature T3 and higher than the temperature T4. In this case, since the final temperature is higher than the temperature T4 which is the melting point of the decomposition gas G2, the decomposition gas G2 is liquefied but not solidified. By contrast, since the final temperature is lower than the temperature T3 which is the melting point of the decomposition gas G1, the decomposition gas G1 is liquefied and then further solidified. Therefore, it can be found that the decomposition gas G1 is solidified in the case without the application of the basic idea in which the decomposition gas G1 and the decomposition gas G2 are collectively cooled to the final temperature lower than the temperature T3 and higher than the temperature T4.

By contrast, discuss a case (first stage) with the application of the basic idea in which the decomposition gas G1 and the decomposition gas G2 are collectively cooled first to the intermediate temperature lower than the temperature T1 and higher than the temperature T2. In this case, the decomposition gas G1 is liquefied but not solidified since the intermediate temperature is lower than the temperature T1 which is the boiling point of the decomposition gas G1, and is higher than the temperature T3 which is the melting point of the decomposition gas G1.

Therefore, at this first stage, the liquefied decomposition gas G1 can be collected. That is, the decomposition gas G1 can be collected in a liquid state before solidified.

And, the decomposition gas G2 is maintained in a gaseous state (gas) since the intermediate temperature is higher than the temperature T2 which is the boiling point of the decomposition gas G2.

Next, discuss a case (second stage) in which the decomposition gas G2 in the gaseous state is cooled from the intermediate temperature to the final temperature lower than the temperature T3 and higher than the temperature T4. In this case, although the final temperature is lower than the temperature T3 which is the melting point of the decomposition gas G1, the liquefied decomposition gas G1 has been already collected and removed at the above-described first stage. Thus, the decomposition gas G1 is not solidified. On the other hand, the decomposition gas G2 is liquefied but not solidified since the final temperature is lower than the temperature T2 which is the boiling point of the decomposition gas G2, and is higher than the temperature T4 which is the melting point of the decomposition gas G2.

Therefore, at this second stage, the liquefied decomposition gas G2 can be collected. That is, the decomposition gas G2 can also be collected in a liquid state before solidified.

As described above, according to the basic idea having the first stage and the second stage, the decomposition gas G1 and the decomposition gas G2 are not collectively cooled to the final temperature, but only the decomposition gas G1 is liquefied and collected by cooling the decomposition gas G1 and the decomposition gas G2 to the intermediate temperature higher than the final temperature, and then, the decomposition gas G2 not liquefied at the intermediate temperature can be liquefied and collected by cooling it to the final temperature. That is, according to the basic idea, by using a difference in the boiling point between the decomposition gas G1 and the decomposition gas G2, the decomposition gas G1 and the decomposition gas G2 can be separately liquefied and collected without being solidified. With this, according to the basic idea, as a result of avoiding the solidification of the decomposition gas G1 and the decomposition gas G2, the clogging in the gas passage due to the deposition of the solidified gas can be suppressed. From this, according to the basic idea, the failure of the continuous operation of the recycle system due to the clogging in the gas passage can be prevented, and, as a result, the decrease in the utilization rate of the recycle system can be suppressed.

Next, the conceptual structure of the recycle system achieving the above-described basic idea will be explained.

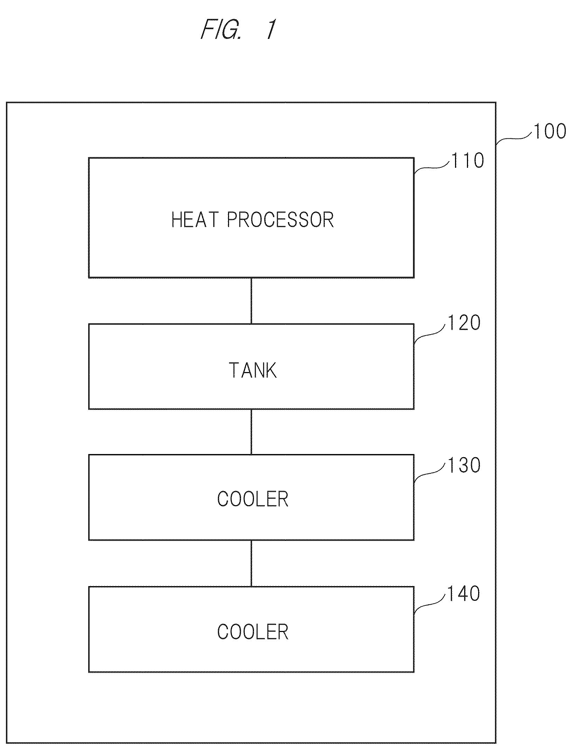

FIG. 1 is a block diagram for explaining a conceptual structure of a recycle system 100.

In FIG. 1, the recycle system 100 includes a heat processor 110, a tank 120, a cooler 130, and a cooler 140.

The heat processor 110 is, for example, an apparatus configured to thermally decompose a resin configuring a plastic product. To this heat processor 110, the tank 120 for storing a decomposition product obtained by thermal decomposition is connected. From the heat processor 110, a decomposition product made of a melt (liquid), gas (decomposition gas), solid, or the like generated by thermally decomposing the resin is discharged.

Here, the solid and the melt are cooled, and then, discharged and collected into the tank 120. On the other hand, to the tank 120, the decomposition gas is also discharged. This decomposition gas can be collected as gas, but becomes difficult to be handled because of having low density and large volume. Therefore, the decomposition gas is desirably collected as liquid. Thus, the recycle system 100 is provided with a cooler for liquefying the decomposition gas discharged to the tank 120.

Here, the cooler provided to the recycle system is made of the cooler 130 and the cooler 140. Therefore, the basic idea can be achieved. That is, according to the recycle system 100, the high-boiling-point gas and the low-boiling-point gas are not collectively cooled to the final temperature. First, only the high-boiling-point gas is liquefied and collected by cooling, at the cooler 130, the high-boiling-point gas and the low-boiling-point gas to the intermediate temperature higher than the final temperature, and then, the low-boiling-point gas not liquefied at the intermediate temperature can be liquefied and collected by cooling it, at the cooler 140, to the final temperature. That is, according to the recycle system 100, by using the difference in the boiling temperature between the high-boiling-point gas and the low-boiling-point gas, the high-boiling-point gas and the low-boiling-point gas can be separately liquefied and collected without being solidified.

With this, according to the recycle system 100, as a result of avoiding the solidification of the high-boiling-point gas and the low-boiling-point gas, the clogging in the gas passage due to the deposition of the solidified gas can be suppressed. From this, according to the recycle system 100, the failure of the continuous operation of the recycle system 100 due to clogging in the gas passage can be prevented, and, as a result, the decrease in the utilization rate of the recycle system 100 can be suppressed.

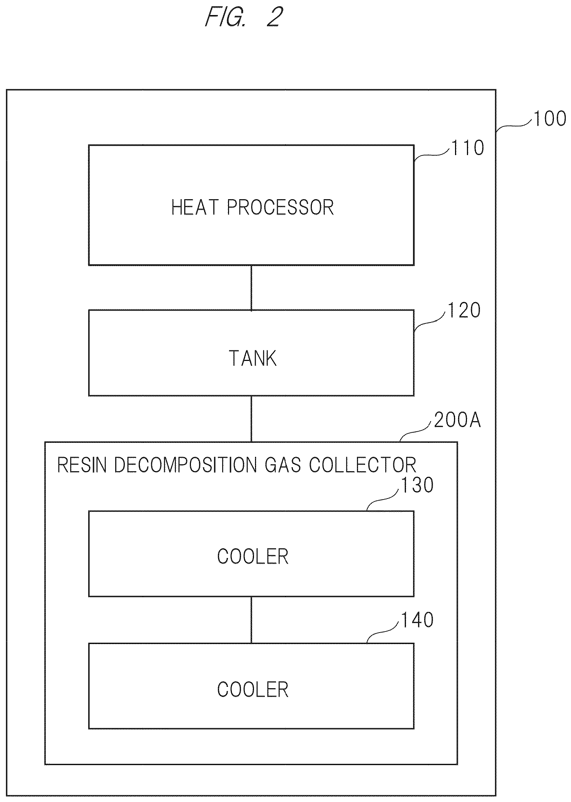

In the above-described recycle system 100, as illustrated in FIG. 2, the cooler 130 and the cooler 140 can be assembled to a resin decomposition gas collector 200A. In this case, the high-boiling-point gas and the low-boiling-point gas generated at the heat processor 110 are discharged to the tank 120, and then, are supplied from the tank 120 to the resin decomposition gas collector 200A. For example, the high-boiling-point gas and the low-boiling-point gas are cooled to the intermediate temperature when being supplied from the tank 120 to the cooler 130. As a result, the liquefied high-boiling-point gas is collected from the cooler 130. On the other hand, the low-boiling-point gas not liquefied at the cooler 130 is supplied in a gaseous state from the cooler 130 to the cooler 140. Then, at the cooler 140, the low-boiling-point gas is liquefied when being cooled from the intermediate temperature to the final temperature, and the liquefied low-boiling-point gas is collected.

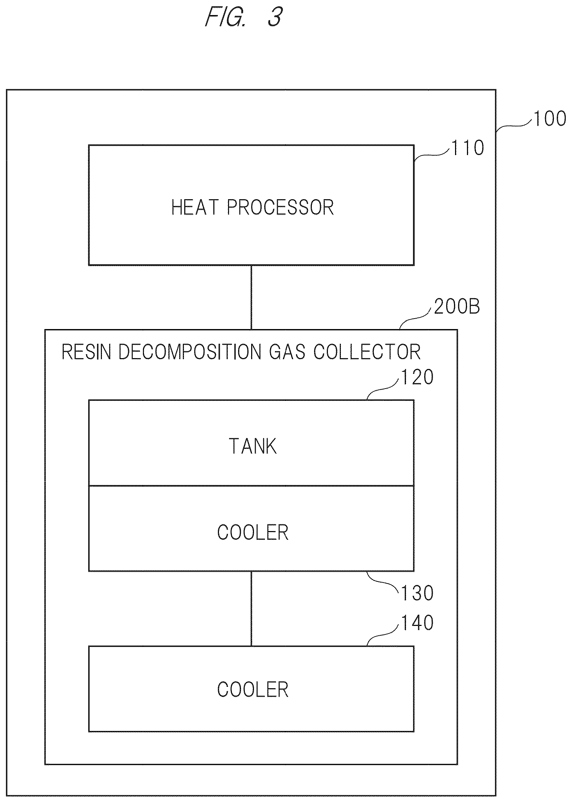

Also, in the recycle system 100, as illustrated in FIG. 3, the cooler 130 and the cooler 140 can be also assembled to a resin decomposition gas collector 200B. As different from the resin decomposition gas collector 200A illustrated in FIG. 2, this resin decomposition gas collector 200B includes the tank 120 as a component, and the cooler 130 is configured to cool this tank 120. In this case, the high-boiling-point gas and the low-boiling-point gas generated at the heat processor 110 are discharged to the tank 120. Here, the tank 120 is cooled to the intermediate temperature by the cooler 130. As a result, the high-boiling-point gas and the low-boiling-point gas supplied to the tank 120 are cooled at the tank 120 to the intermediate temperature. Therefore, the liquefied high-boiling-point gas is collected from the tank 120. On the other hand, the low-boiling-point gas not liquefied at the tank 120 is supplied in a gaseous state from the tank 120 to the cooler 140. Then, at the cooler 140, the low-boiling-point gas is liquefied when being cooled from the intermediate temperature to the final temperature. Therefore, the low-boiling-point gas is collected from the cooler 140.

As described above, it is considered that the recycle system 100 achieving the basic idea has the mode illustrated in FIG. 2 and the mode illustrated in FIG. 3. Thus, an embodied mode of a recycle system 100 adopting the mode illustrated in FIG. 2 will be explained below, and a recycle system 100 adopting the mode illustrated in FIG. 3 will be explained as a first modification example.

### <Embodied Mode>

### <<Structure of Recycle System>>

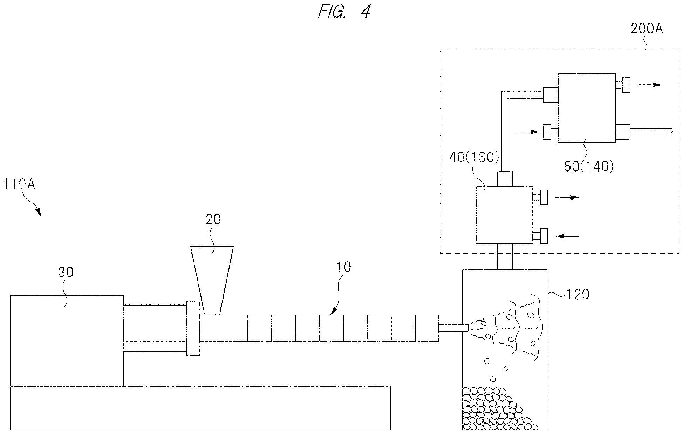

FIG. 4 is a diagram illustrating the structure of the recycle system in the embodied mode.

In FIG. 4, the recycle system in the embodied mode includes an extruder 110A, the tank 120, and the resin decomposition gas collector 200A. Here, in the embodied mode, the extruder 110A is used as one example of the heat processor 110 illustrated in FIG. 2.

The extruder 110A includes a cylinder 10, a hopper 20, and a rotation driving mechanism 30. The cylinder 10 is made of a plurality of cylinder blocks. Inside this cylinder 10, a screw is arranged, and the screw is configured to be rotatable by the rotation driving mechanism 30. Also, the tank 120 is attached to a tip portion of the cylinder 10. This tank 120 is connected to the resin decomposition gas collector 200A via, for example, piping. Furthermore, the cylinder 10 is connected to the hopper 20, and this hopper 20 functions as a raw material supply port for supplying a raw material into the cylinder 10.

In the extruder 110A configured as described above, when the raw material is supplied to the hopper 20, the raw material is fed into the cylinder 10. The raw material fed into the cylinder 10 is kneaded by the screw provided inside the cylinder 10. Specifically, the raw material fed into the cylinder 10 is kneaded by the screw rotated by the rotation driving mechanism 30, and is transferred as a kneaded product from the upstream toward the downstream of the cylinder 10.

Here, the kneaded product is decomposed into a decomposition product of a solid, liquid, or gas. The decomposition product flows into the tank 120 attached to the tip portion of the cylinder 10, and then, the gas included in the decomposition product is exhausted from the tank 120.

The resin decomposition gas collector 200A includes a heat exchanger 40 and a heat exchanger 50.

Here, one example of the cooler 130 illustrated in FIG. 2 is the heat exchanger 40, and one example of the cooler 140 illustrated in FIG. 2 is the heat exchanger 50.

The heat exchanger 40 is connected to the tank 120, and is configured to allow the gas discharged from the extruder 110A to the tank 120 to flow thereinto. And, the heat exchanger 40 is configured so as to cool the gas flowing from the tank 120 to the intermediate temperature.

On the other hand, the heat exchanger 50 is connected to the heat exchanger 40, and is configured to allow the gas discharged from the heat exchanger 40 to flow thereinto. And, the heat exchanger 50 is configured so as to cool the gas discharged from the heat exchanger 40 to the final temperature lower than the intermediate temperature.

For example, the heat exchanger 40 is configured to be able to perform cooling at the intermediate temperature which is higher than the temperature T2 and equal to or lower than the temperature T1. As a result, the heat exchanger 40 is configured to liquefy and collect only a high-boiling-point gas when the high-boiling-point gas with a boiling point equal to or higher than the temperature T1 and a low-boiling-point gas with a boiling point of the temperature T2 lower than the temperature T1 are introduced thereto.

On the other hand, the heat exchanger 50 is configured to be able to perform cooling at the final temperature equal to or lower than the temperature T2, and is configured liquefy and collect the low-boiling-point gas when the low-boiling-point gas is introduced thereto.

The recycle system is configured as described above.

### <<Operation of Recycle System>>

Next, the operation of the recycle system is described.

In FIG. 4, the resin is introduced to the hopper 20 of the extruder 110A. When the resin is introduced to the hopper 20, the resin is fed into the cylinder 10. The resin fed into the cylinder 10 is kneaded by the screw provided inside the cylinder 10. Specifically, the raw material fed into the cylinder 10 is kneaded by the screw rotated by the rotation driving mechanism 30, and is transferred as the kneaded product from the upstream toward the downstream of the cylinder 10.

From the extruder 110A, the decomposition product formed of the melt (liquid), the gaseous state (gas), the solid, or the like generated by thermally decomposing the resin is discharged to the tank 120.

Here, the decomposition gas includes gases of a plurality of types. For example, in order to explain the technical significance of the technical idea to be simply understood, it is assumed that the gases of the plurality of types are two types that are a high-boiling-point gas A(g) and a low-boiling-point gas B(g). These high-boiling-point gas A(g) and low-boiling-point gas B(g) flow from the tank 120 into the resin decomposition gas collector 200A.

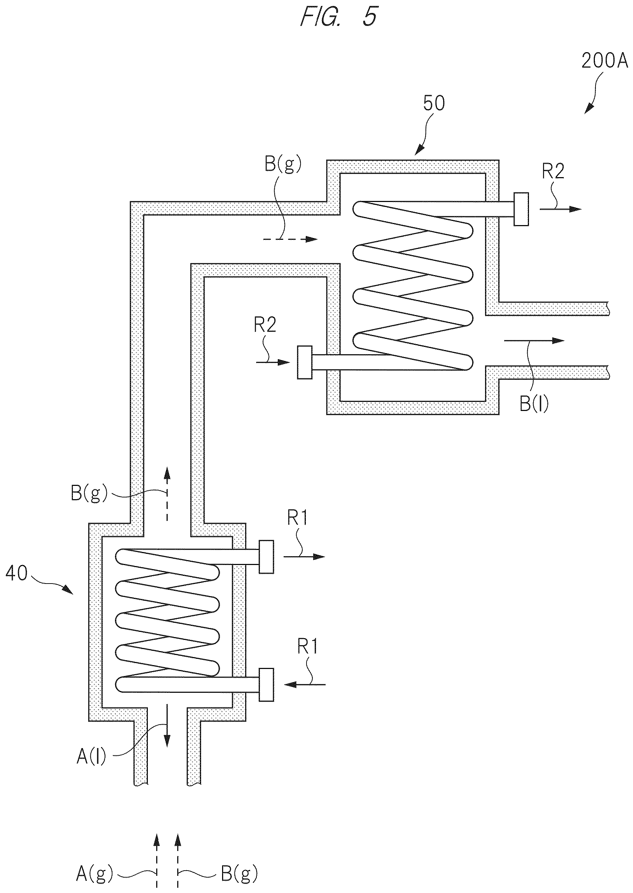

FIG. 5 is a diagram illustrating an example of structure of the resin decomposition gas collector 200A.

As illustrated in FIG. 5, the high-boiling-point gas A(g) and the low-boiling-point gas B(g) flown into the resin decomposition gas collector 200A are supplied to the heat exchanger 40. At the heat exchanger 40, by a circulating coolant R1, the high-boiling-point gas A(g) and the low-boiling-point gas B(g) are cooled to the intermediate temperature. Here, the intermediate temperature is a temperature lower than the boiling point of the high-boiling-point gas A(g) and higher than the boiling point of the low-boiling-point gas B(g). Also, the intermediate temperature is a temperature higher than the melting point of the high-boiling-point gas A(g). Therefore, when the high-boiling-point gas A(g) is cooled to the intermediate temperature, the high-boiling-point gas A(g) is liquefied to become a liquefied gas A(l), but is not solidified. This liquefied gas A(l) is stored inside the tank 120 by, for example, being dropped to the tank 120 by gravity. As a result, the liquefied gas A(l) can be collected from the tank 120.

Note that the resin decomposition gas collector 200A may not be configured such that the liquefied gas A(l) is dropped to the tank 120 and is collected, and may be configured such that the liquefied gas A(l) is stored in a storage different from the tank 120 and is collected.

When the low-boiling-point gas B(g) is cooled to the intermediate temperature, the low-boiling-point gas B(g) is not liquefied, and remains in the gaseous state (gas form). Then, as illustrated in FIG. 5, the low-boiling-point gas B(g) cooled to the intermediate temperature is discharged from the heat exchanger 40, and then, is supplied to the heat exchanger 50.

At the heat exchanger 50, by a circulating coolant R2, the low-boiling-point gas B(g)is cooled to the final temperature. Here, the final temperature is a temperature lower than the intermediate temperature, lower than the boiling point of the low-boiling-point gas B(g) and higher than the melting point of the low-boiling-point gas B(g). Therefore, when the low-boiling-point gas B(g) is cooled to the final temperature, the low-boiling-point gas B(g) is liquefied to become a liquefied gas B(l), but is not solidified. This liquefied gas B(l) is discharged from the heat exchanger 50, and then, is collected. By the operation of the recycle system as described above, the method of collecting the resin decomposition gas in the embodied mode is achieved.

The above-described method of collecting the resin decomposition gas is summarized as follows.

That is, the method of collecting the resin decomposition gas includes a step (a) of liquefying a high-boiling-point gas among the high-boiling-point gas and a low-boiling-point gas generated by thermally decomposing the resin; and a step (b) of liquefying the low-boiling-point gas after the step (a).

Furthermore, the boiling point of the high-boiling-point gas is equal to or higher than the temperature T1, and the boiling point of the low-boiling-point gas is the temperature T2 which is lower than the temperature T1. Here, in the step (a), the high-boiling point gas is liquefied and collected by introducing the high-boiling-point gas and the low-boiling-point gas to the heat exchanger 40 configured to be able to perform cooling at the intermediate temperature higher than the temperature T2 and equal to or lower than the temperature T1. On the other hand, in the step (b), the low-boiling-point gas is liquefied and collected by introducing the low-boiling-point gas to the heat exchanger 50 configured to be able to perform cooling at the final temperature equal to or lower than the temperature T2.

In the method of collecting the resin decomposition gas achieved by the operation of the above-described recycle system, the liquefied high-boiling-point gas and the liquefied low-boiling-point gas are collected. As a result, for example, when the liquefied high-boiling-point gas and the liquefied low-boiling-point gas are reused, the resin as the raw material for the product can be synthesized again.

However, a mode of reusing the liquefied high-boiling-point gas and the liquefied low-boiling-point gas includes not only the mode of synthesizing the resin as the raw material for the product again but also a mode of, for example, recycling the liquefied high-boiling-point gas and the liquefied low-boiling-point gas as fuel. In this case, the method of collecting the resin decomposition gas achieved by the operation of the above-described recycle system can be interpreted as a method of producing recyclable liquefied gas. That is, it can be said that the technical idea in the embodied mode has one aspect as the method of collecting the resin decomposition gas and another aspect as the method of producing the recyclable liquefied gas.

### <<Specific Examples of Resin>>

The resin to be thermally decomposed in the recycle system is not particularly limited, and, for example, can be polyethylene resin, polypropylene resin, polyamide-6 resin, polycarbonate resin, polyethylene terephthalate resin, or polybutylene terephthalate resin.

Typical resins will be exemplified below, and an application example thereof to the technical idea will be explained.

### 1. Polycarbonate Resin

When the resin as the target for the thermal decomposition is a polycarbonate resin, water is added as a reaction aid or additive, the thermal decomposition process is performed at the extruder 110A. Here, the internal temperature (temperature of the cylinder 10) of the extruder 110A is, for example, 260°C to 320°C.

By the thermal decomposition of polycarbonate resin, bisphenol A (melting point: 159°C, boiling point: 220°C) and phenol (melting point: 40.5°C, boiling point: 182°C) are generated. Here, the high-boiling-point gas is bisphenol A, and the low-boiling-point gas is phenol. Note that the gas discharged from the extruder 110A includes the bisphenol A and the phenol described above and unreacted water (melting point: 0°C, boiling point: 100°C).

Therefore, for example, while bisphenol A is liquefied by setting the intermediate temperature which is the cooling temperature at the heat exchanger 40 to be equal to or higher than 182°C and lower than 220°C, phenol is liquefied by setting the final temperature which is the cooling temperature at the heat exchanger 50 to be lower than 182°C. Here, for example, by setting the temperature of the heat exchanger 50 to be equal to or higher than 100°C and lower than 182°C, phenol can be collected as the liquid while water (water vapor) can be discharged to the atmosphere.

### 2. Polyamide-6 Resin

When the resin as the target for the thermal decomposition is a polyamide-6 resin, water is added as a reaction aid or additive, and the thermal decomposition process is performed at the extruder 110A. Here, the internal temperature of the extruder 110A is, for example, 230°C to 350°C.

By the thermal decomposition of polyamide-6 resin, caprolactam (melting point: 69°C, boiling point: 267°C) is generated. Here, the gas discharged from the extruder 110A includes caprolactam and unreacted water (melting point: 0°C, boiling point: 100°C). Here, the high-boiling-point gas is caprolactam, and the low-boiling-point gas is water vapor.

Therefore, for example, caprolactam is liquefied by setting the intermediate temperature which is the cooling temperature at the heat exchanger 40 to be equal to or higher than 100°C and lower than 267°C while water vapor is liquefied by setting the final temperature which is the cooling temperature at the heat exchanger 50 to be lower than 100°C.

### 3. Polyethylene Terephthalate Resin

When the resin as the target for the thermal decomposition is a polyethylene terephthalate resin, water or ethylene glycol is added as a reaction aid or additive, and the thermal decomposition process is performed at the extruder 110A. Here, the internal temperature of the extruder 110A is, for example, 255°C to 320°C.

When the ethylene glycol is added to polyethylene terephthalate resin, by the thermal decomposition of polyethylene terephthalate resin, bis (2-hydroxyethyl) terephthalate (melting point: 106°C, boiling point: 317°C or higher) is generated. Here, the gas discharged from the extruder 110A includes bis (2-hydroxyethyl) terephthalate and unreacted ethylene glycol (melting point: -10°C, boiling point: 244°C). Here, the high-boiling-point gas is bis (2-hydroxyethyl) terephthalate, and the low-boiling-point gas is ethylene glycol.

Therefore, bis (2-hydroxyethyl) terephthalate is liquefied by setting the intermediate temperature which is the cooling temperature at the heat exchanger 40 to be equal to or higher than 244°C and lower than 317°C while ethylene glycol is liquefied by setting the final temperature which is the cooling temperature at the heat exchanger 50 to be lower than 244°C.

### 4. Polyolefin Resin (Chain Hydrocarbon Resin)

When the resin as the target for the thermal decomposition is a polyolefin resin, the thermal decomposition process is performed at the extruder 110A. Here, the internal temperature of the extruder 110A is, for example, 300°C to 430°C.

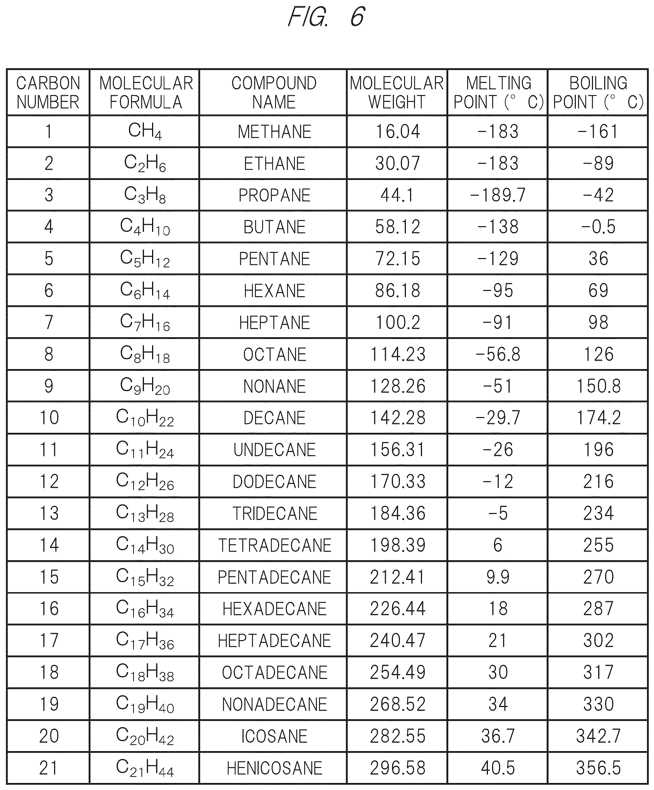

By this thermal decomposition, polyolefin resin is decomposed to hydrocarbon compounds of many types different in the carbon number. As a hydrocarbon compound to be gasified, for example, any of hydrocarbon compounds illustrated in FIG. 6 can be exemplified. The melting points and the boiling points of these hydrocarbon compounds are also illustrated.

In this case, the gas discharged from the extruder 110A is gas including hydrocarbon compounds of many types with carbon numbers of 1 to 21 illustrated in FIG. 6. Depending on the temperature condition, a hydrocarbon compound with a large carbon number is not gasified inside the extruder 110A but becomes in a melting state, and is discharged from the tip portion of the extruder 110A into the tank 120, and then, is collected therein.

Then, the generated gas is introduced to the heat exchanger 40. Here, any hydrocarbon compound can be selected as the high-boiling-point gas to be liquified, and can be determined in consideration of a control temperature at the next heat exchanger 50. For example, when the control temperature at the heat exchanger 50 is set to 15°C (room temperature), the gas to be cooled at the heat exchanger 50 is deposited as a solid matter at the heat exchanger 50 when having a melting point equal to or higher than 15°C. Therefore, in this case, if a hydrocarbon with a carbon number higher than pentadecane with a hydrocarbon number of 15 (melting point: 9.9°C) (hydrocarbon with a carbon number equal to or higher than 16) is included, a solid matter is adhered inside the heat exchanger 50. If the process continues, efficiency of the thermal decomposition process decreases. When the adhesion of the solid matter further proceeds and is deposited, there is the possibility of the clogging of the gas passage, which results in difficulty in the continuation of the thermal decomposition process.

Therefore, under the above-described condition, it is desirable to set a hydrocarbon with a carbon number of 16 (hexadecane, chemical formula: C₁₆H₃₄) as the high-boiling-point gas to be liquified at the heat exchanger 40. In this case, since the hexadecane is liquefied at the heat exchanger 40, the hexadecane is not included in the gas introduced to the heat exchanger 50. Therefore, even if the control temperature of the heat exchanger 50 is set to 15°C, a gas with a melting point equal to or higher than 15°C is not included, and therefore, there is no gas to be solidified inside the heat exchanger 50, and the occurrence of the above-described failure can be suppressed.

When the process is performed as described above, it is only required to set the control temperature of the heat exchanger 40 to, for example, a temperature equal to or higher than its melting point and lower than its boiling point (equal to or higher than 18°C and lower than 287°C). Here, in this thermal decomposition process, compounds with similar molecular weight, melting point and boiling point are included. Thus, it is desirable to efficiently liquefy a target compound while not to include a compound with a boiling point lower than that of that target compound as much as possible, thereby not including a necessary compound. Therefore, the control temperature of the heat exchanger 40 in the thermal decomposition under the above-described condition is desirably set to a range of about 230°C to 260°C.

### <<Feature Point in Embodied Mode>>

Next, a feature point in the embodied mode will be explained.

The feature point in the embodied mode is that the technique of collecting the resin decomposition gas includes a first stage of collectively cooling the high-boiling-point gas and the low-boiling-point gas to the intermediate temperature and a second stage of cooling the low-boiling-point gas in the gaseous state from the intermediate temperature to the final temperature.

According to this feature point, the high-boiling-point gas and the low-boiling-point gas are not collectively cooled to the final temperature, but the high-boiling-point gas and the low-boiling-point gas are cooled to the intermediate temperature higher than the final temperature, and only the high-boiling-point gas is liquified and collected, and then, the low-boiling-point gas not liquefied at the intermediate temperature is cooled to the final temperature, and can be liquified and collected.

That is, according to the feature point, by using the difference in the boiling point between the high-boiling-point gas and the low-boiling-point gas, the high-boiling-point gas and the low-boiling-point gas can be liquified and collected separately without not being solidified. Therefore, according to the feature point, the solidification of the high-boiling-point gas and the low-boiling-point gas can be avoided, and, as a result, the clogging in the gas passage due to the deposition of the solidified gas can be suppressed.

Therefore, according to the feature point, the failure of the continuous operation of the recycle system due to the clogging in the gas passage can be prevented. As a result, according to the feature point, the decrease in the utilization rate of the recycle system can be suppressed.

### <First Modification Example>

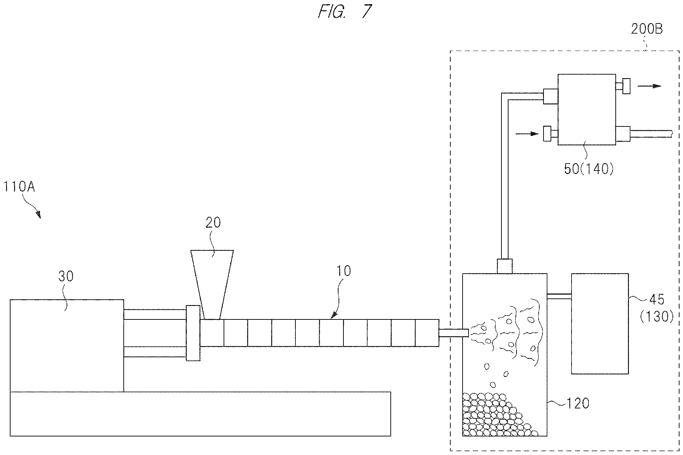

In the above-described embodied mode, for example, as illustrated in FIG. 4, the resin decomposition gas collector 200A is configured so as to include the heat exchanger 40 and the heat exchanger 50. That is, the resin decomposition gas collector 200A in the embodied mode has the structure in which the basic idea illustrated in FIG. 1 is embodied by the mode illustrated in FIG. 2. However, the basic idea illustrated in FIG. 1 can be embodied also as the mode illustrated in FIG. 3. This structure is the present first modification example, and FIG. 7 is a diagram illustrating a recycle system including the resin decomposition gas collector 200B in the present first modification example.

In FIG. 7, the resin decomposition gas collector 200B includes the tank 120 connected to the tip portion (downstream) of the extruder 110A, a cooler 45 for cooling the inside of the tank 120, and the heat exchanger 50 connected to the tank 120. Specifically, the cooler 45 is configured to be able to perform cooling, at the intermediate temperature, the tank 120 provided at a discharge port of the heat processor (extruder 110A) that thermally decomposes the resin, the discharge port discharging the high-boiling-point gas and the low-boiling-point gas. On the other hand, the heat exchanger 50 is configured to be connectable to the tank 120.

According to the resin decomposition gas collector 200B configured as described above, since the tank 120 is cooled to the intermediate temperature by the cooler 45, only the high-boiling-point gas among the high-boiling-point gas and the low-boiling-point gas discharged from the extruder 110A into the tank 120 is liquefied. On the other hand, the low-boiling-point gas in the gaseous state is introduced from the tank 120 to the heat exchanger 50, and is cooled to the final temperature inside this heat exchanger 50 and is liquefied. In this manner, the basic idea illustrated in FIG. 1 can be embodied also as the resin decomposition gas collector 200B in the present first modification example illustrated in FIG. 7.

### <Second Modification Example>

In the above-described embodied mode, for example, as illustrated in FIG. 4, the resin decomposition gas collector 200A is connected to the tank 120. However, the present invention is not limited to this structure, and the resin decomposition gas collector 200A may be connected to a vent portion provided to the extruder 110A.

For example, in the structure in which the resin decomposition gas collector 200A is connected to the tank 120, the high-boiling-point gas liquefied at the heat exchanger 40 drops into the tank 120 and is accumulated. Here, when the resin is a polyolefin resin, the high-boiling-point gas to be liquefied at the heat exchanger 40 is a gas with a large carbon number. Therefore, when such a gas with a large carbon number is desired to be used, the structure in which the resin decomposition gas collector 200A is connected to the tank 120 is desirable.

By contrast, in order to improve an yield of obtaining a gas with a carbon number smaller than the gas with the large carbon number, it is desirable to apply not the structure in which the resin decomposition gas collector 200A is connected to the tank 120 but the structure in which the resin decomposition gas collector 200A is connected to the vent portion as described in the present second modification example. This is because, in this structure, the gas with the large carbon number liquefied at the heat exchanger 40 is not fed to the tank 120 but is returned into the extruder 110A via the vent portion and is decomposed to the gas with the small carbon number again inside the extruder 110A.

In the foregoing, the invention made by the inventors of the present application has been concretely described on the basis of the embodiments. However, it is needless to say that the present invention is not limited to the foregoing embodiments, and various modifications can be made within the scope of the present invention.

### EXPLANATION OF REFERENCE CHARACTERS

- 10: cylinder
- 20: hopper
- 30: rotation driving mechanism
- 40: heat exchanger
- 50: heat exchanger
- 100: recycle system
- 110: heat processor
- 110A: extruder
- 120: tank
- 130: cooler
- 140: cooler
- 200A: resin decomposition gas collector
- 200B: resin decomposition gas collector
- A(g): high-boiling-point gas
- A(l): liquefied gas
- B(g): low-boiling-point gas
- B(l): liquefied gas
- R1: coolant
- R2: coolant

## Claims

1. A method of collecting a resin decomposition gas, comprising steps of:
(a) liquefying a first decomposition gas among the first decomposition gas and a second decomposition gas generated by thermally decomposing a resin; and
(b) after the step (a), liquefying the second decomposition gas.

2. The method of collecting the resin decomposition gas according to claim 1,
wherein a boiling point of the first decomposition gas is a temperature higher than a first temperature,
a boiling point of the second decomposition gas is a second temperature lower than the first temperature,
at the step (a), the first decomposition gas is liquefied and collected by introducing the first decomposition gas and the second decomposition gas to a first cooler configured to be able to perform cooling at a temperature higher than the second temperature and equal to or lower than the first temperature, and
at the step (b), the second decomposition gas is liquefied and collected by introducing the second decomposition gas to a second cooler configured to be able to perform cooling at a temperature lower than the second temperature.

3. The method of collecting the resin decomposition gas according to claim 2,
wherein the first cooler is made of a first heat exchanger, and
the second cooler is made of a second heat exchanger.

4. The method of collecting the resin decomposition gas according to claim 2,
wherein the first cooler is configured to be able to perform cooling, at the first temperature, a tank provided at a discharge port of a heat processor that thermally decomposes the resin, the discharge port discharging the first decomposition gas and the second decomposition gas, and
the second cooler is made of a heat exchanger connected to the tank.

5. The method of collecting the resin decomposition gas according to claim 4,
wherein the heat processor is an extruder.

6. A method of collecting a resin decomposition gas, comprising a step of:
separately liquefying and collecting a plurality of gases by using a difference in a boiling point among the plurality of gases generated by thermally decomposing a resin.

7. A method of producing a recyclable liquefied gas, comprising steps of:
(a) liquefying a first decomposition gas among the first decomposition gas and a second decomposition gas generated by thermally decomposing a resin; and
(b) after the step (a), liquefying the second decomposition gas.

8. The method of producing the recyclable liquefied gas according to claim 7,
wherein a boiling point of the first decomposition gas is a temperature higher than a first temperature,
a boiling point of the second decomposition gas is a second temperature lower than the first temperature,
at the step (a), the first decomposition gas is liquefied and collected by introducing the first decomposition gas and the second decomposition gas to a first cooler configured to be able to perform cooling at a temperature higher than the second temperature and lower than the first temperature, and
at the step (b), the second decomposition gas is liquefied and collected by introducing the second decomposition gas to a second cooler configured to be able to perform cooling at a temperature lower than the second temperature.

9. The method of producing the recyclable liquefied gas according to claim 8,
wherein the first cooler is made of a first heat exchanger, and
the second cooler is made of a second heat exchanger.

10. The method of producing the recyclable liquefied gas according to claim 8,
wherein the first cooler is configured to be able to perform cooling, at the first temperature, a tank provided at a discharge port of a heat processor that thermally decomposes the resin, the discharge port discharging the first decomposition gas and the second decomposition gas, and
the second cooler is made of a heat exchanger connected to the tank.

11. The method of producing the recyclable liquefied gas according to claim 10,
wherein the heat processor is an extruder.

12. A method of producing a recyclable liquefied gas, comprising a step of:
separately liquefying and collecting a plurality of gases by using a difference in a boiling point among the plurality of gases generated by thermally decomposing a resin.

13. A resin decomposition gas collector, comprising:
a first cooler configured to liquify and collect a first decomposition gas among the first decomposition gas and a second decomposition gas generated by thermally decomposing a resin; and
a second cooler configured to liquify and collect the second decomposition gas.

14. The resin decomposition gas collector according to claim 13,
wherein a boiling point of the first decomposition gas is a temperature higher than a first temperature,
a boiling point of the second decomposition gas is a second temperature lower than the first temperature,
the first cooler is configured to be able to perform cooling at an intermediate temperature higher than the second temperature and lower than the first temperature,
the first cooler is configured to liquify and collect the first decomposition gas by cooling the first decomposition gas and the second decomposition gas to the intermediated temperature,
the second cooler is configured to be able to perform cooling at a final temperature lower than the second temperature, and
the second cooler is configured to liquify and collect the second decomposition gas by cooling the second decomposition gas to the final temperature.

15. The resin decomposition gas collector according to claim 14,
wherein the second cooler is configured to liquefy and collect the second decomposition gas by introducing the second decomposition gas discharged from the first cooler to the second cooler and cooling the second decomposition gas to the final temperature.

16. The resin decomposition gas collector according to claim 13,
wherein the first cooler is made of a first heat exchanger, and
the second cooler is made of a second heat exchanger.

17. The resin decomposition gas collector according to claim 13,
wherein the first cooler is configured to be able to perform cooling, at the first temperature, a tank provided at a discharge port of a heat processor that thermally decomposes the resin, the discharge port discharging the first decomposition gas and the second decomposition gas, and
the second cooler is made of a heat exchanger connected to the tank.

18. The resin decomposition gas collector according to claim 17,
wherein the heat processor is an extruder.

19. A resin decomposition gas collector, comprising:
a plurality of liquefying/collecting portions configured to separately liquefy and collect a plurality of gases by using a difference in a boiling point among the plurality of gases generated by thermally decomposing a resin.

20. The resin decomposition gas collector according to claim 19,
wherein the plurality of gases are recyclable gases.
